(19) Europäisches Patentamt
European Patent Office
Office européen des brevets

(11) Publication number: **0 372 541 A1**

(12) **EUROPEAN PATENT APPLICATION**

(21) Application number: **89122544.3**

(22) Date of filing: **07.12.89**

(51) Int. Cl.$^5$: **A61K 37/02, //(A61K37/02, 31:557)**

(30) Priority: **09.12.88 US 282096**

(43) Date of publication of application: **13.06.90 Bulletin 90/24**

(84) Designated Contracting States: **AT BE CH DE ES FR GB GR IT LI LU NL SE**

(71) Applicant: **G.D. Searle & Co.**
**P.O. Box 5110**
**Chicago Illinois 60680(US)**

(72) Inventor: **Stanford, Mark Moran**
**33 Kings Cross Drive**
**Lincolnshire IL 60015(US)**

(74) Representative: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

# (54) Method for imparting immunosuppression.

(57) A method for imparting immunosuppression in a patient in need thereof is performed by administering an immunosuppressive agent containing cyclosporine and administering an E-type prostaglandin in an immunosuppressive-agent-enhancing effective dose.

EP 0 372 541 A1

Xerox Copy Centre

# METHOD FOR IMPARTING IMMUNOSUPRESSION

## BACKGROUND OF THE INVENTION

Prostaglandins are a family of biologically potent lipid acids. They have been categorized into several main classes which are designated by letters and distinguished by substitutions on the cyclopentane ring. The main classes are further subdivided in accord with the number of double bonds in the side chains. This further subdivision is indicated by subscripts on the acronym abbreviation for the particular prostaglandin.

It has been known for some time that prostaglandins by themselves exhibit immunosuppressive activity. While the immunosuppressive activity of the prostaglandins by themselves has been accepted, the relatively high concentration of prostaglandins required to exhibit the immunosuppressive activity has been sufficiently high that the chronic use of prostaglandins as immunosuppressants has not been possible. It has been well known that prostaglandins exhibit deleterious side effects such as diarrhea, abdominal pain, menstrual irregularities and fevers when administered in high dosage concentrations.

The combination of certain prostaglandins with known immunosuppressive agents has also been evaluated. Prostacyclin was evaluated in a human study of eight patients by Leithner, et al. (reported in Prostaglandins, Vol. 22 No. 5, Nov. 1981) in combination with prednisolone and azathioprine to determine the immunosuppressive activity with respect to kidney transplant recipients. Prostacyclin is a $PGI_2$ prostaglandin. It was administered acutely by infusion into a central vein of a patient after the patient had exhibited rejection of the transplant.

Iloprost, a prostacyclin or $PGI_2$ analog, was evaluated for its activity as an immunosuppressant in a study of cardiac allografts in rats. Rowles, et al. compared the immunosuppressant activity of the combination of iloprost and cyclosporine (CsA) with a combination of cyclosporine and prednisolone (Transplantation, Vol. 42 No. 1, July 1986). The dosages of the prostaglandin and CSA were administered parenterally. Although the combination of cyclosporine with prednisolone exhibited better immunosuppressive activity than the combination of cyclosporine and iloprost, Rowles, et al. suggested that iloprost could be useful to replace prednisolone.The dose of iloprost was 0.72 mg per kg given daily by osmotic pump.

U.S. Patent 4,452,794 of Kort, et al. describes the use of prostaglandins as transplant rejection medicines. The studies therein are of a $PGE_1$ analog which was evaluated in rats that had undergone heart transplants. The rats had initially received azathioprine or prednisolone then on the fifth day post-transplantation had been orally administered a mixture of azathioprine or prednisolone and the $PGE_1$ prostaglandin analog which mixture contained 1 mg per kg of the $PGE_1$ analog. Kort, et al. claimed that the use of the $PGE_1$ analog enhanced the immunosuppressive activity of the corticosteroid (prednisolone) or the mercaptopurine (azathioprine) that were used as the main immunosuppressants.

In a similarly conducted study Ryffel, et al. (Transplantation Proceedings, Vol. XVIII, No. 4, August 1986) conducted a study to determine the immunosuppressive activity of a $PGE_2$ analog in combination with the immunosuppressant agent cyclosporine in rats undergoing renal allografts. While Ryffel, et al. found that the $PGE_2$ analog did abolish cyclosporine nephrotoxicity at the 10 $\mu$g per kg dosage administered subcutaneously by minipump, they did not recommend the use of the $PGE_2$ analog as it inhibited the enteral absorption of cyclosporine. They stated that the decreased cyclosporine bioavailability accompanied by reduced immunosuppression precludes $PGE_2$ being used clinically to prevent cyclosporine nephrotoxicity.

Although there have been teachings of immunosuppressant properties of prostaglandins as noted above, prostaglandins of the E and I series have not been used clinically with any significant success as immunosuppressants. The teachings to date have suggested that the required doses in man to achieve meaningful immunosuppression would be too large to be tolerable.

Cyclosporine is a cyclic polypeptide which consists of eleven amino acids and is produced as a metabolite by the fungus species Tolypocladium inflatum Gams. Cyclosporine is widely used as an immunosuppressant agent and is particularly useful in aiding transplant recipients from rejecting the transplanted organ. An unfortunate side effect of the use of cyclosporine is that it can exhibit acute nephrotoxicity. The use of cyclosporine can cause significant kidney damage for patients receiving organ transplants, including to the transplanted kidney itself if the procedure is a kidney transplant. The term cyclosporine is used herein to include cyclosporine and its analogs, specifically cyclosporin A (CsA). Cyclosporin A is commercially available from Sandoz, Inc.

## SUMMARY OF THE INVENTION

The present invention is directed to a method for imparting immunosuppression to a patient in need thereof by administering a cyclosporine-containing immunosuppressive agent and administering an E-type prostaglandin in an immunosuppressive-agent-enhancing effective dose. In particular, the present invention is directed to a method for imparting immunosuppression in a patient in need thereof by administering an immunosuppressive agent containing cyclosporine in an immunosuppressive effective dose and administering an E-type prostaglandin in an immunosuppressive-agent-enhancing effective dose.

More particularly, the invention herein is directed to a method of imparting immunosuppression in a patient in need thereof by administering an immunosuppressive agent containing cyclosporine in a dose that is less than or equal to an immunosuppressive-effective dose of the cyclosporine containing immunosuppressive agent were administered alone and administering a prostaglandin of the E-series, such as a $PGE_1$, $PGE_2$ or $PGE_3$, in an immunosuppressive-agent-enhancing effective dose.

## BRIEF DESCRIPTION OF THE DRAWINGS

The method that is the subject of the invention herein will be better understood with reference to the accompanying drawings wherein:

Figure 1 is a graph of results from a clinical study showing serum creatinine vs time. The changes in serum creatinine are shown for three months of treatment and three months of withdrawal. Renal transplantation was performed at time 0. $PGE_1$ prostaglandin: solid squares. Placebo: open circles.

Figure 2 is a graph of results from the same human clinical study showing creatinine clearance vs time.The changes in creatinine clearance are shown for three months of treatment and one month of withdrawal. Renal transplantation was performed at time 0. $PGE_1$ prostaglandin: solid squares. Placebo: open circles.

Figure 3 is a graph of results from the same human clinical study showing mean cyclosporine dosage vs time. Mean doses are shown in four week segments. $PGE_1$ prostaglandin: broad hash marks. Placebo: narrow hash marks.

Figure 4 is a graph of results from the same human clinical study showing trough cyclosporine blood levels by HPLC (high pressure liquid chromatography). Changes in trough levels vs time are shown for sixteen weeks. Renal transplantation was performed at the beginning of week #1. $PGE_1$ prostaglandin: solid circles. Placebo: open circles.

Figure 5 is a graph of results from the same human clinical study showing acute cyclosporine nephrotoxicity. The incidence rate of first episodes is shown for sixteen weeks. Renal transplantation was performed at the beginning of week #1. $PGE_1$ prostaglandin: broad hash marks. Placebo: narrow hash marks.

Figure 6 is a graph showing acute rejection data from the human clinical study. The incidence rate of first episodes is shown for sixteen weeks. Renal transplantation was performed at the beginning of week #1. Treatment with the $PGE_1$ prostaglandin ceased at week #12. $PGE_1$ prostaglandin: broad hash marks. Placebo: narrow hash marks.

Figure 7 is a graph showing mixed lymphocyte responses (MLR) for certain prostaglandins. The percent inhibition of the mixed lymphocyte response is shown for a five-log change in drug concentration. $PGE_1$ prostaglandin: solid squares. $PGE_2$ prostaglandin: open squares. $PGE_1$ prostaglandin free acid: solid circles.

Figure 8 is a graph showing mixed lymphocyte responses for certain prostaglandins and ethanol. The percent inhibition of the mixed lymphocyte response is shown for three concentrations of each of three prostaglandins along with the associated concentration of ethanol present as a diluent. $PGE_1$ prostaglandin: solid squares. $PGE_1$ prostaglandin free acid: solid circles. $PGE_2$ prostaglandin: open circles. Ethanol: open squares.

Figure 9 is a graph of MLR response to doubling of immunosuppressant concentration. The percent inhibition of the mixed lymphocyte response is shown for a 1X and 2X concentration of $PGE_1$ prostaglandin free acid (solid triangles), cyclosporine (open circles) and methylprednisolone (open squares).

Figure 10 is a graph showing the immunosuppressive effect of $PGE_1$ prostaglandin free acid. A larger than additive effect is shown when the free acid is added to either cyclosporine (solid squares (cyclosporine alone shown in open circles)) or methylprednisolone (solid circles (methylprednisolone alone shown in open squares)).

## DETAILED DESCRIPTION OF THE INVENTION

The invention herein is a method for imparting immunosuppression in a patient in need thereof by administering an immunosuppressive agent containing cyclosporine and also administering a prostaglandin of the E-series (PGE) in an immunosuppressive-agent-enhancing effective dose. One benefit derived from the method herein is that the administration of the PGE enhances the immunosuppressive activity of the cyclosporine-containing immunosuppressive agent such that the cyclosporine-containing immunosuppressive agent can be used in a concentration less than the concentration which would be used if the cyclosporine-containing immunosuppressive agent were used without the prostaglandin. A second benefit derived from the method herein is that the administration of the PGE enhances the immunosuppressive activity of the cyclosporine such that if the cyclosporine-containing immunosuppressive agent is administered in a dosage equivalent to the dosage in which it would be administered without the prostaglandin, a greater immunosuppressive effect would be achieved which could lessen damage to the kidney due to rejection by the patient's autoimmune system. In view of the known deleterious side effects of the use of cyclosporine, the method herein can diminish the likelihood and/or occurrence of such deleterious side effects of the cyclosporine.

The terms "prostaglandin" and/or its accepted acronym "PG" or, as more appropriately used herein for the E-series prostaglandins, "PGE," are used herein to refer to the naturally occurring or man-made E-series prostaglandins and their analogs and derivatives.

It has been found herein that $E_1$ prostaglandin shown by the following formula I

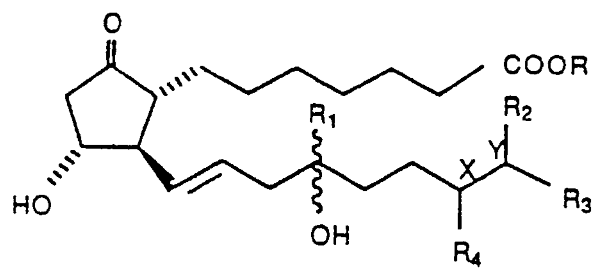

I

$E_2$ prostaglandins shown by the following formula II

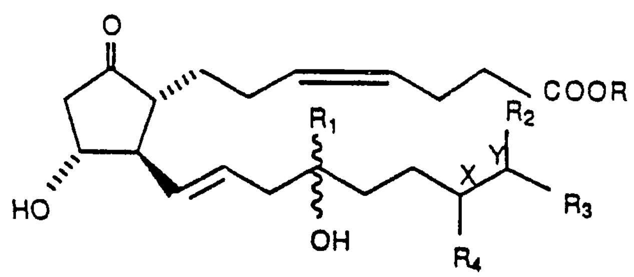

II

and $E_3$ prostaglandins shown by the following formula III

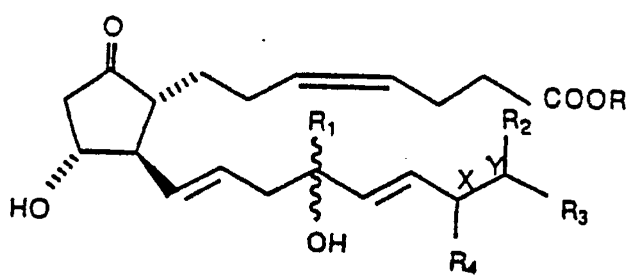

III

wherein R represents hydrogen or lower alkyl having 1 to 6 carbon atoms, $R_1$ represents hydrogen, vinyl or lower alkyl having 1 to 4 carbon atoms and the wavy line represents R or S stereochemistry; $R_2$, $R_3$, and $R_4$ are hydrogen or lower alkyl having 1 to 4 carbon atoms or $R_2$ and $R_3$ together with carbon Y form a cycloalkenyl having 4 to 6 carbon atoms or $R_3$ or $R_4$ together with carbons X and Y form a cycloalkenyl

having 4 to 6 carbons.

By lower alkyl is meant straight or branched chain alkyl such as methyl, ethyl, propyl, isopropyl, butyl, secondary butyl or tertiary butyl, pentyl, or hexyl with the indicated limitation of the number of carbon atoms. The bond between carbon X and carbon Y can be saturated or unsaturated.

With regard to the illustrated structures, the dashed line indicates the grouping being behind the plane of the paper that the structure is drawn on and the solid, blackened triangular shape indicates that the group is in front of the plane of the paper.

The prostaglandins used in the method of this invention can be prepared by known reaction schemes such as by the methods taught in U. S. Patents 3,965,143, 4,271,314, and 4,683,328. The individual isomers can be obtained by chromatographic separation.

Regardless of the route of administration selected, the prostaglandin, as used in the method herein, can be formulated into pharmaceutically acceptable dosage forms by conventional methods known to the pharmaceutical art.

The prostaglandin compounds can be administered in such oral unit dosage forms as tablets, capsules, pills, powders, or granules. They also can be administered intraperitoneally, subcutaneously, or intramuscularly, using forms known in the pharmaceutical art. In general, the preferred form of administration is oral. An effective but non-toxic quantity of the prostaglandin is employed in the treatment. The dosage regimen for the method of this invention is selected in accordance with a variety of factors including the type, age, weight, sex, and medical condition of the patient, the particular organ transplant the patient has undergone, the route of administration and the particular prostaglandin employed. Dosages of the prostaglandin useful in the method herein are in the range of up to 120 µg/kg per day with a preferred range of 2.5 µg/kg to 40 µg/kg per day. Greater or lesser dosages can be used, depending upon the above-noted factors.

The term "immunosuppressive-agent" as used herein means any agent, whether a single compound, composition, combination, and/or co-administered regimen of such a compound, composition and combination, which is administered in a therapeutic regimen for suppressing the autoimmune response of a person in need of such therapy. For example, the term "immunosuppressive agent" includes cyclosporine which is administered with adjunct steroid therapy such as a corticosteroid (e.g., prednisone, methylprednisolone and the like) or a mercaptopurine (e.g., azathioprine).

The term "immunosuppressive-effective dosage" as used herein means, with respect to the immunosuppressive agent, the dose sufficient for imparting the requisite suppression of the autoimmune response in the patient in need of such therapy. The specific dose is dependent upon the same factors noted above with respect to the dosage of prostaglandin. The specific dosage is well within the skill of the prescribing physician. The Physician's Desk Reference, 42nd Edition (1988) lists the daily single dose of cyclosporine that is continued postoperatively for one to two weeks as 14-18 mg/kg/day. This dosage is decreased by 5% per week to a maintenance level of 5-10 mg/kg/day. The dosages can be adjusted upwardly or downwardly to achieve specific plasma levels or whole blood levels. Doses as low as 3 mg/kg/day have also been used.

The method herein is performed by administering the cyclosporine-containing immunosuppressive agent to the patient in need of such therapy and administering the E-series prostaglandin to the patient. The two administration steps can be performed in any sequence and with the lapse of time between administration steps to accomplish the above-noted daily dosages. The sequencing and timing of the performance of the steps of the method herein are well within the skill of a physician prescribing the immunosuppressive regimen.

The efficacy of the method herein has been demonstrated through in vitro tests as well as an in vivo clinical trial. A randomized, double-blind, placebo controlled study was designed: (1) to determine the safety and efficacy of a $PGE_1$ prostaglandin in reducing cyclosporine-induced nephrotoxicity in renal transplant recipients; and (2) to examine the effects of the $PGE_1$ prostaglandin on the incidence rate of clinically important events such as acute transplant rejection.

The PGE, prostaglandin evaluated was misoprostol, ((±) methyl 11α, 16-dihydroxy-16-methyl-9-oxoprost-13E-en-1-oate). Subjects received the $PGE_1$ prostaglandin in dosages of 200 µg or a placebo four times a day (Q.I.D.) for the first twelve weeks following transplantation. A study population of 77 subjects was evaluated. Efficacy was assessed by a serial comparison of renal function and by measuring the duration and functional severity of transplant rejection, cyclosporine nephrotoxicity, and other episodes of acute renal failure in the $PGE_1$ prostaglandin and the placebo groups. Serial measurements of serum creatinine and creatinine clearance were used to determine trends and differences in renal function between treatment groups.

The subjects in the clinical study were first stratified into two groups according to the source of the

allograft, cadaver or living-related donor. Members of each group were assigned at random in the order of enrollment to receive the $PGE_1$ prostaglandin or the placebo according to a computer-generated assignment schedule prepared prior to the start of the study. The purpose of this approach was to guarantee adequate randomization of subjects as a function of allograft source and probable post-transplantation function.

The administration of the prostaglandin began with the first dose of cyclosporine. The subjects received the .prostaglandin Q.I.D. given 30-60 minutes p.c. (after a meal) and h.s. (at night). The prostaglandin initially could be administered via a nasogastric tube. Following discharge of the patient from the hospital. the patient began to administer the placebo or prostaglandin and recording the dosing in a diary. The test article dosing remained one tablet Q.I D. continuing the 30-60 minute p.c and h.s. schedule.

Hematology and chemistry profiles were obtained on the first and seventh post-transplantation days and again after four weeks. A chemistry profile was obtained at eight and twelve weeks. A urinalysis was obtained on the first post-operative day and again after four weeks.

Twenty-four hour creatinine clearance measurements were obtained on post-operative days one, three, and seven, then weekly for three weeks and at four week intervals thereafter until the study was terminated.

Serum creatinine measurements were obtained on postoperative days 1, 3 and 7, and then at weeks 4, 8, 12 and 16. (Note days 1 and 3 are not shown in the figs.)

Measurement of trough cyclosporine levels were required on days one, three, five and seven; twice during each of weeks two, three and four; and at least once during weeks eight, twelve and sixteen.

Acute renal failure was defined as the presence of any of the following:

1) Any change in renal function which prompted the principal investigator to perform a diagnostic maneuver (e.g., measure of serum creatinine concentration or urinary sodium concentration) or therapeutic maneuver (e.g., give intravenous fluids) to investigate or treat the change.

2) A rise in serum creatinine concentration from a stable base line of at least 0.2 mg/dl/day for two days or 0.3 mg/dl in one day.

3) A fall in urine output to less than an average of 25 ml per hour for more than 24 hours.

After twelve weeks of treatment and four additional weeks of observation, the subject underwent a physical examination and a complete medical history was reviewed. The following laboratory tests were also obtained: hematology, PT/PTT, chemistry, urinalysis. creatinine clearance, cyclosporine level and serum beta HCG (where appropriate). In addition, any other measurements of creatinine concentration or clearance obtained during the post-treatment period were recorded.

The results of the human clinical study indicated that the prostaglandin was associated with: (1) improved renal function as shown in Figures 1 and 2; (2) no interference with cyclosporine absorption as shown in figures 3 and 4; (3) no increase in cyclosporine nephrotoxicity as shown in Figure 5; and a marked reduction in the incidence rate of acute transplant rejection and therefore enhanced immunosuppression as shown in Figure 6.

The results of the clinical trial are expressed in the following Table 1:

TABLE 1

| Test | Medicine | 1 Month | 2 Months | 3 Months |
|---|---|---|---|---|
| Creatinine (mg/dl) | Placebo<br>$PGE_1$ | 2.2 ± 0.2<br>1.6 ± 0.2 | 1.9 ± 0.2<br>1.4 ± 0.1 | 1.8 ± 0.1<br>1.4 ± 0.1 |
| Creatinine Clearance (ml/min/1.73m2) | Placebo<br>$PGE_1$ | 60 ± 5<br>73 ± 5 | 66 ± 5<br>77 ± 6 | 64 ± 5<br>81 ± 5 |
| Acute Rejection (first episode/mo) | Placebo<br>$PGE_1$ | 10<br>3 | 4<br>2 | 2<br>2 |

As shown in Table I, the acute rejection was significantly reduced during the three months of treatment with the $PGE_1$ prostaglandin ($p = 0.015$). It can be concluded from the study that the $PGE_1$ prostaglandin improved renal function in cyclosporine-treated renal transplant recipients and reduced the frequency of acute transplant rejection.

In vitro studies of the prostaglandins and their effect on cyclosporine have also been conducted. In the studies, a $PGE_1$ prostaglandin commonly referred to as misoprostol and having the structure shown by the

general formula I wherein R and $R_1$ are methyl, $R_2$, $R_3$ and $R_4$ are all hydrogen, its free acid (misoprostol free acid) and a $PGE_2$ Prostaglandin commonly referred to as enisoprost which has the structure shown by the general formula II wherein R and $R_1$ are both methyl and $R_2$, $R_3$ and $R_4$ are all hydrogen with the X-Y bond being saturated, were evaluated. These three prostaglandins were studied to determine their effect on the inhibition of the mixed lymphocyte response (MLR).

The mixed lymphocyte response is a clinically useful predictor of the immunosuppressive activity of drugs.The mixed lymphocyte response assay data shown below and in Figures 7-10 were obtained with the following method. A stimulator panel of Epstein-Barr virus (EBV) transformed continuous lymphoblastoid cell lines was employed to magnify responses. There were equal numbers of four cell lines with eight different HLA-DR antigens (two per cell line). Stimulator cells were irradiated (13,000 rad) before suspension in a modified RPMI medium. Responder lymphocytes were prepared from heparinized recipient blood by density gradient separation before suspension in glutamine with antibiotic-supplemented RPMI medium containing 10% autologous serum. Equal numbers of responder and stimulator cells ($1x\ 10^5$ in 0.1 ml) were incubated with 0.05 ml of medium (control) or varying concentrations of the three prostaglandin analogs (misoprostol, enisoprost and misoprostol-free acid) or methylprednisolone or cyclosporine or combinations of these agents.

Following incubation at 37°C and 5% $CO_2$ for five days, one microcurie of tritiated thymidine was added and cell cultures incubated for six hours. Cells were then harvested. MLR responses for the three different prostaglandin analogs were then compared for several different concentrations (Figure 7). The effects of the prostaglandins were also compared to those of the control ethanol diluent (Figure 8).

Increasing inhibition of the mixed lymphocyte response (MLR) reflects immunosuppressive activity. As shown in Figure 7, there is a clear dose-response demonstration of inhibition for all three prostaglandin analogs. The effect of the ethanol diluent was negligible (Figure 8). The effect on the MLR of joint or combined administration of the prostaglandin and cyclosporine or methylprednisolone is shown in Figures 9 and 10. As demonstrated, through a comparison of the data in Figures 9 and 10, there is enhanced inhibition in the presence of the prostaglandin with either of the other drugs.

These studies show that there was clear evidence of enhancement of immunosuppressive activity between misoprostol free acid (prostaglandin analog) and both methylprednisolone and cyclosporine. These in vitro data corroborate and extend the in vivo data previously shown.

The use of the method herein can enhance the immunosuppressive effect of an immunosuppressive therapeutic regimen which includes the administration of cyclosporine. The method can thus provide the desired extent of immunosuppression by using a lower dose of the cyclosporine-containing immunosuppressive agent. The method can, therefore, reduce the incidence of acute rejection and reduce the acute nephrotoxicity of cyclosporine. An unpredicted but beneficial aspect of the method described herein is that, despite enhanced immunosuppression with respect to the transplanted organ, the addition of prostaglandins did not increase patient susceptibility to infection during the study.

## Claims

1. Use of an immunosuppressive agent containing cyclosporine; and an E-type prostaglandin in an immunosuppressive-agent-enhancing effective dose for preparing a medicament for imparting immunosuppression in a patient in need thereof.

2. Use according to Claim 1 wherein the immunosuppressive agent comprises cyclosporine.

3. Use according to Claim 1 wherein the immunosuppressive agent comprises cyclosporine in combination with a second immunosuppressive agent.

4. Use according to Claim 3 wherein the second immunosuppressive agent comprises a corticosteroid.

5. Use according to Claim 3 wherein the second immunosuppressive agent comprises azathioprine.

6. Use according to Claim 1 wherein the immunosuppressive agent is used in an amount which is less than an immunosuppressive-effective amount if the immunosuppressive agent were used alone.

7. Use according to Claim 1 wherein the E-type prostaglandin has the structural formula selected from the following:

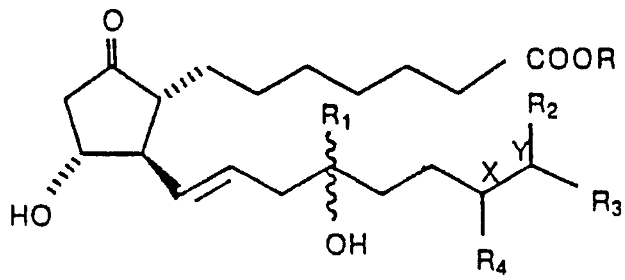

I

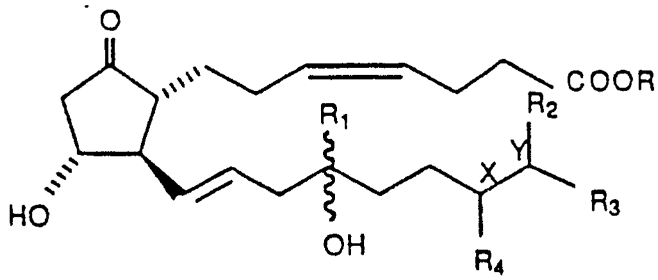

II

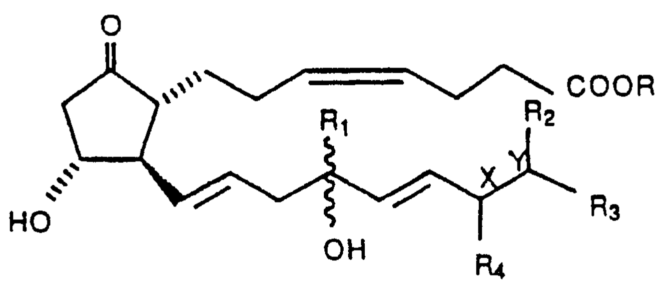

III

wherein R represents hydrogen or lower alkyl having 1 to 6 carbon atoms; $R_1$ represents hydrogen, vinyl or lower alkyl having 1 to 4 carbon atoms and the wavy line represents R or S stereochemistry; $R_2$, $R_3$, and $R_4$ are hydrogen or lower alkyl having 1 to 4 carbon atoms or $R_2$ and $R_3$ together with carbon Y form a cycloalkenyl having 4 to 6 carbon atoms or $R_3$ and $R_4$ together with carbons X and Y form a cycloalkenyl having 4 to 6 carbons and wherein the X-Y bond can be saturated or unsaturated.

8. Use of an immunosuppressive agent which includes cyclosporine in an amount less than an immunosuppressive effective dose when used alone; and
an immunosuppressive-agent-enhancing effective dose of an E-type prostaglandin for preparing a medicament for imparting immunosuppression in an organ transplant recipient.

9. Use according to Claim 8 wherein the immunosuppressive agent comprises cyclosporine.

10. Use according to Claim 8 wherein the immunosuppressive agent comprises cyclosporine in combination with a second immunosuppressive agent.

11. Use according to Claim 10 wherein the second immunosuppressive agent comprises a corticosteroid.

12. Use according to Claim 10 wherein the second immunosuppressive agent comprises azathioprine.

13. Use of a cyclosporine-containing immunosuppressive agent in an amount less than or equal to an immunosuppressive-effective dose when such immunosuppressive agent is used alone; and
an immunosuppressive-agent-enhancing effective dose of an E-type prostaglandin for preparing a medicament for improving renal function in a patient having undergone an organ or tissue transplant.

14. Use according to Claim 13 wherein the immunosuppressive agent comprises cyclosporine.

15. Use according to Claim 13 wherein the immunosuppressive agent comprises cyclosporine in combination with a second immunosuppressive agent.

16. Use according to Claim 15 wherein the second immunosuppressive agent comprises a corticosteroid.

17. Use according to Claim 15 wherein the second immunosuppressive agent comprises azathioprine.

18. Use according to Claim 13 wherein the E-type prostaglandin has a structural formula selected from the following:

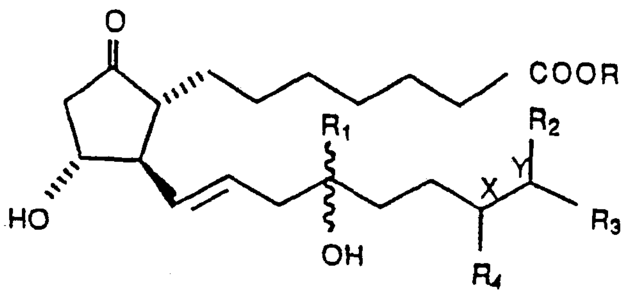

I

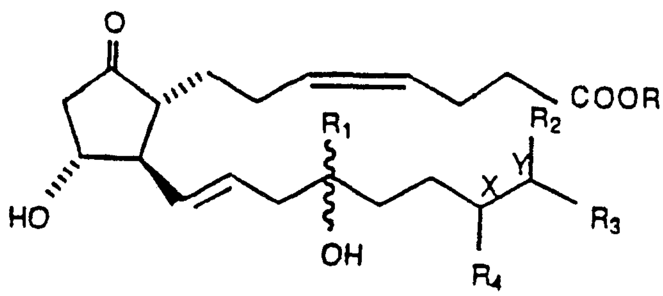

II

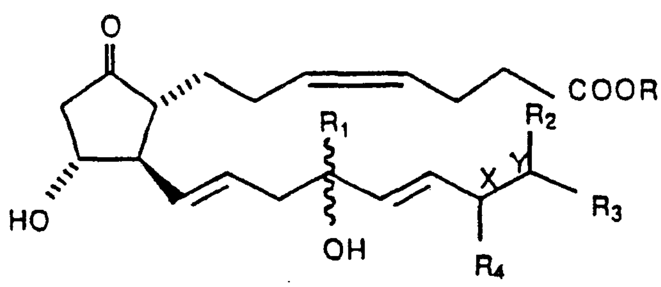

III

wherein R represents hydrogen or lower alkyl having 1 to 6 carbon atoms; $R_1$ represents hydrogen, vinyl or lower alkyl having 1 to 4 carbon atoms and the wavy line represents R or S stereochemistry; $R_2$, $R_3$, and $R_4$ are hydrogen or lower alkyl having 1 to 4 carbon atoms or $R_2$ and $R_3$ together with carbon Y form a cycloalkenyl having 4 to 6 carbon atoms or $R_3$ and $R_4$ together with carbons X and Y form a cycloalkenyl having 4 to 6 carbons and wherein the X-Y bond can be saturated or unsaturated.

19. Use according to Claim 13 wherein the E-type prostaglandin comprises misoprostol.

20. Use according to Claim 13 wherein the E-type prostaglandin comprises enisoprost.

FIGURE 1

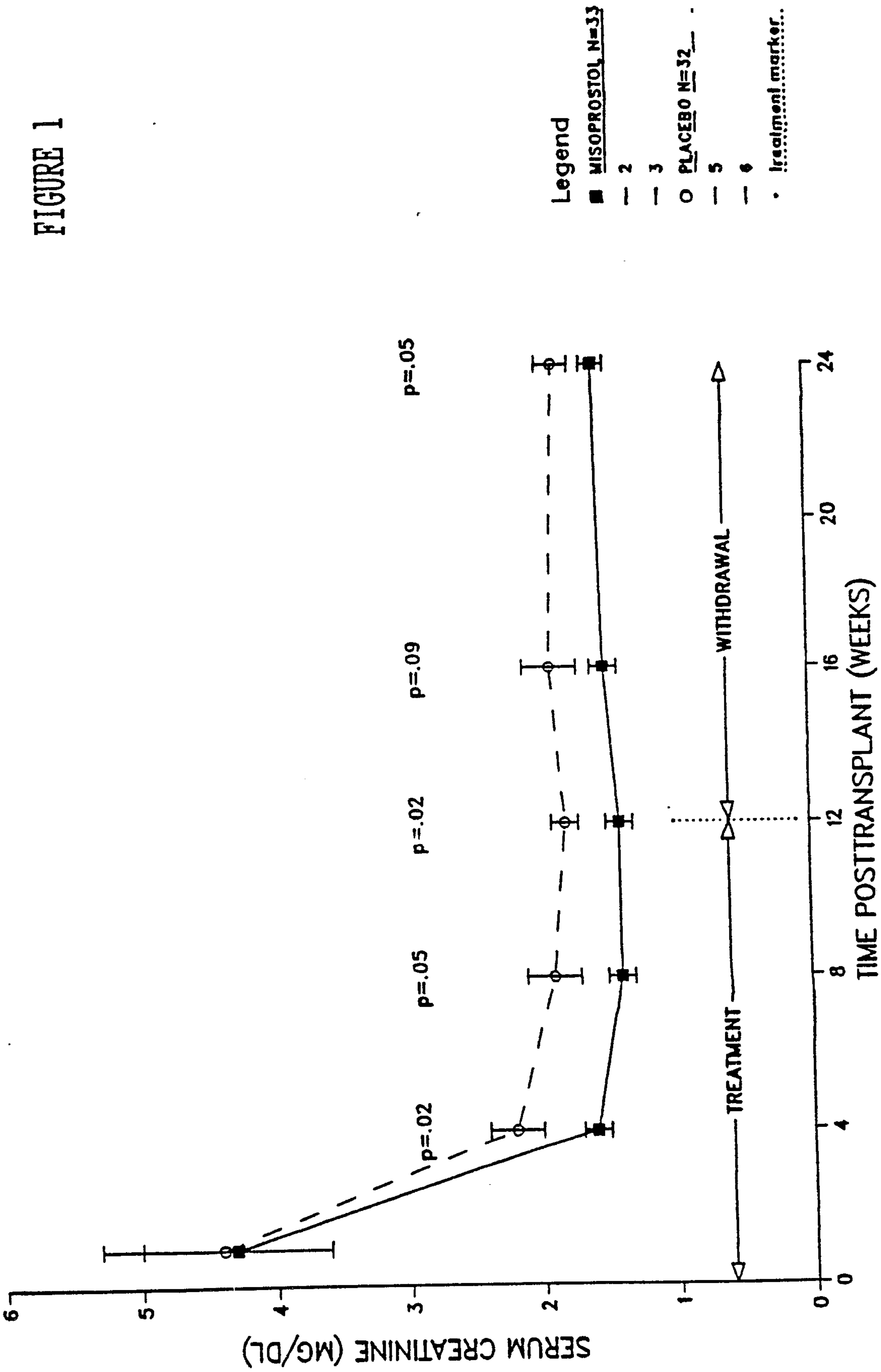

Legend
MISOPROSTOL N=33
2
3
PLACEBO N=32
5
6
treatment marker
p=.02
p=.05
p=.02
p=.09
p=.05
TREATMENT
WITHDRAWAL
SERUM CREATININE (MG/DL)
0
1
2
3
4
5
6
TIME POSTTRANSPLANT (WEEKS)
0
4
8
12
16
20
24

FIGURE 2

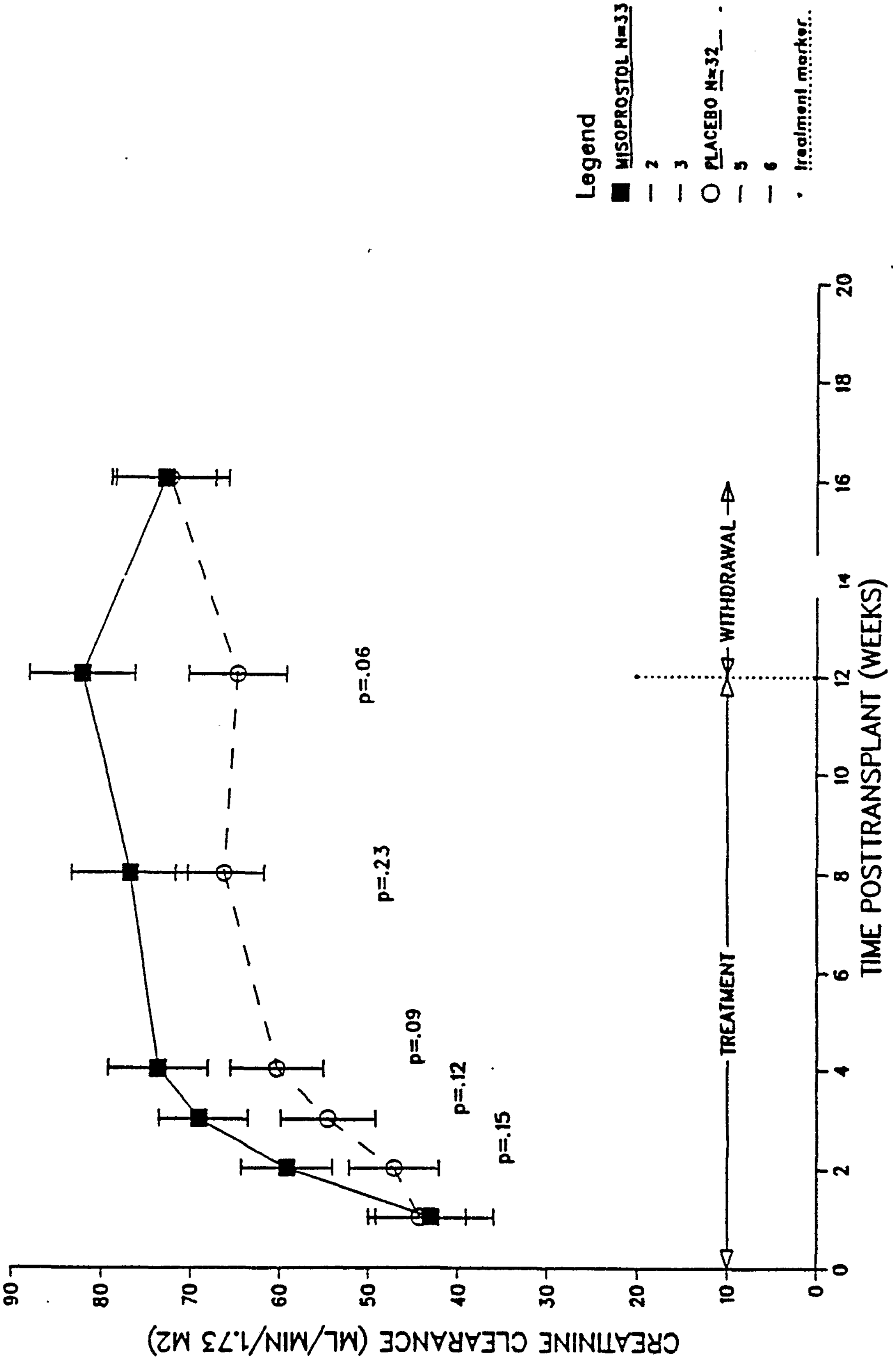
CREATININE CLEARANCE (ML/MIN/1.73 M2)
TIME POSTTRANSPLANT (WEEKS)
0
10
20
30
40
50
60
70
80
90
0
2
4
6
8
10
12
14
16
18
20
p=.15
p=.12
p=.09
p=.23
p=.06
TREATMENT
WITHDRAWAL
Legend
MISOPROSTOL N=33
2
3
PLACEBO N=32
5
6
treatment marker

FIGURE 3

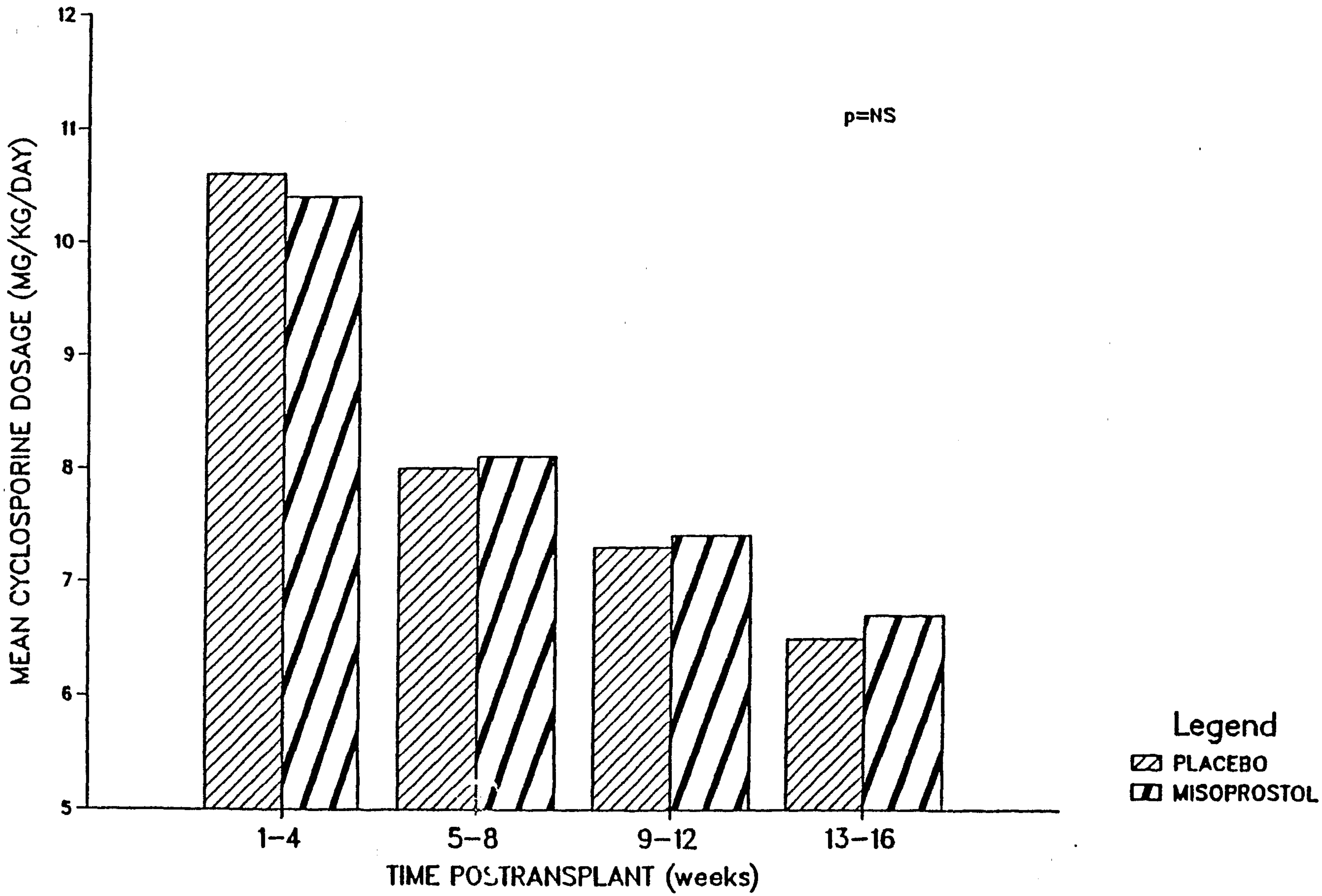
p=NS
12
11
10
9
8
7
6
5
MEAN CYCLOSPORINE DOSAGE (MG/KG/DAY)
1–4
5–8
9–12
13–16
TIME POSTRANSPLANT (weeks)
Legend
PLACEBO
MISOPROSTOL

FIGURE 4

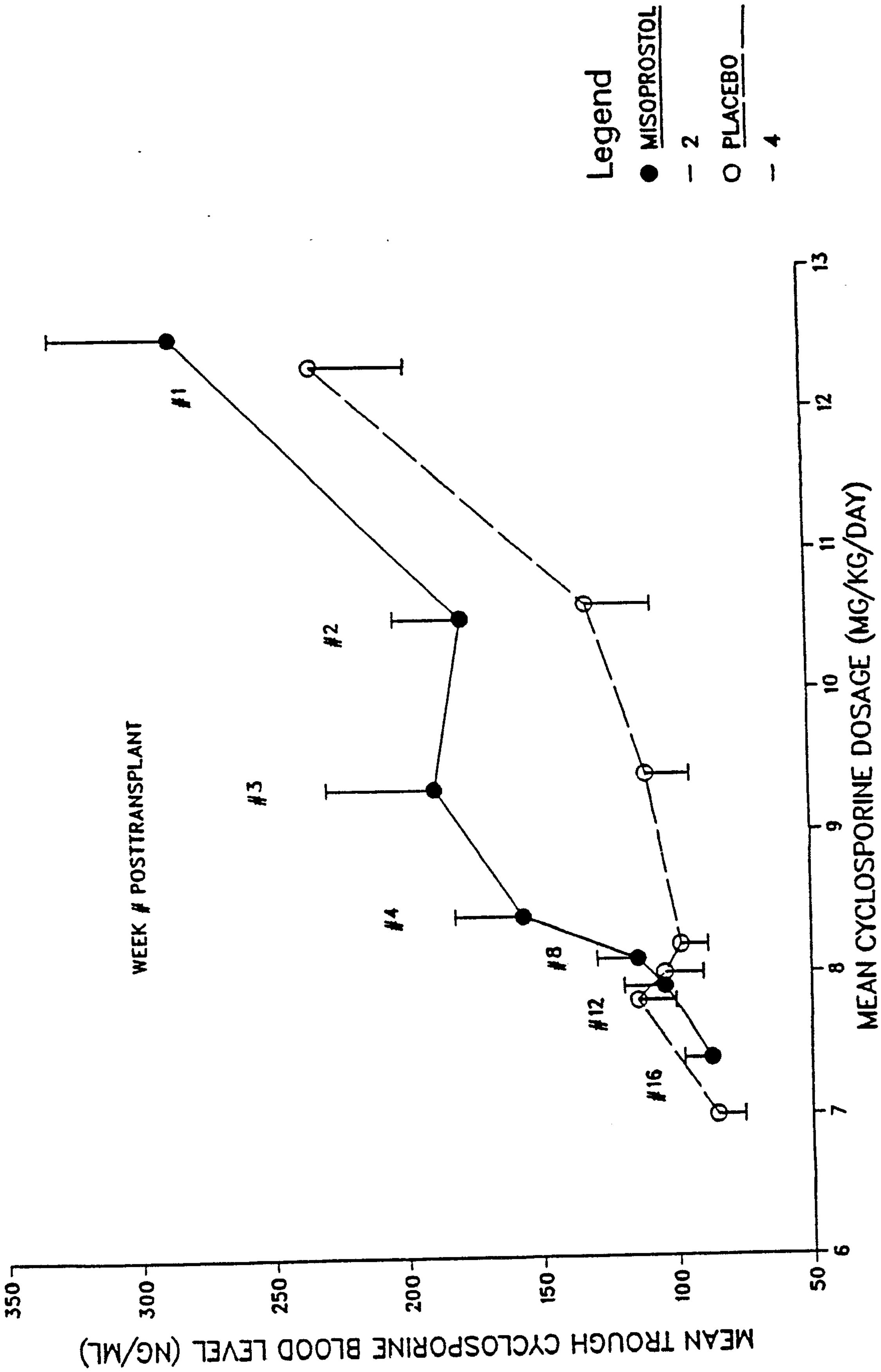
WEEK # POSTTRANSPLANT
#1
#2
#3
#4
#8
#12
#16
Legend
● MISOPROSTOL
— 2
O PLACEBO
— 4
MEAN CYCLOSPORINE DOSAGE (MG/KG/DAY)
MEAN TROUGH CYCLOSPORINE BLOOD LEVEL (NG/ML)
6
7
8
9
10
11
12
13
50
100
150
200
250
300
350

FIGURE 5

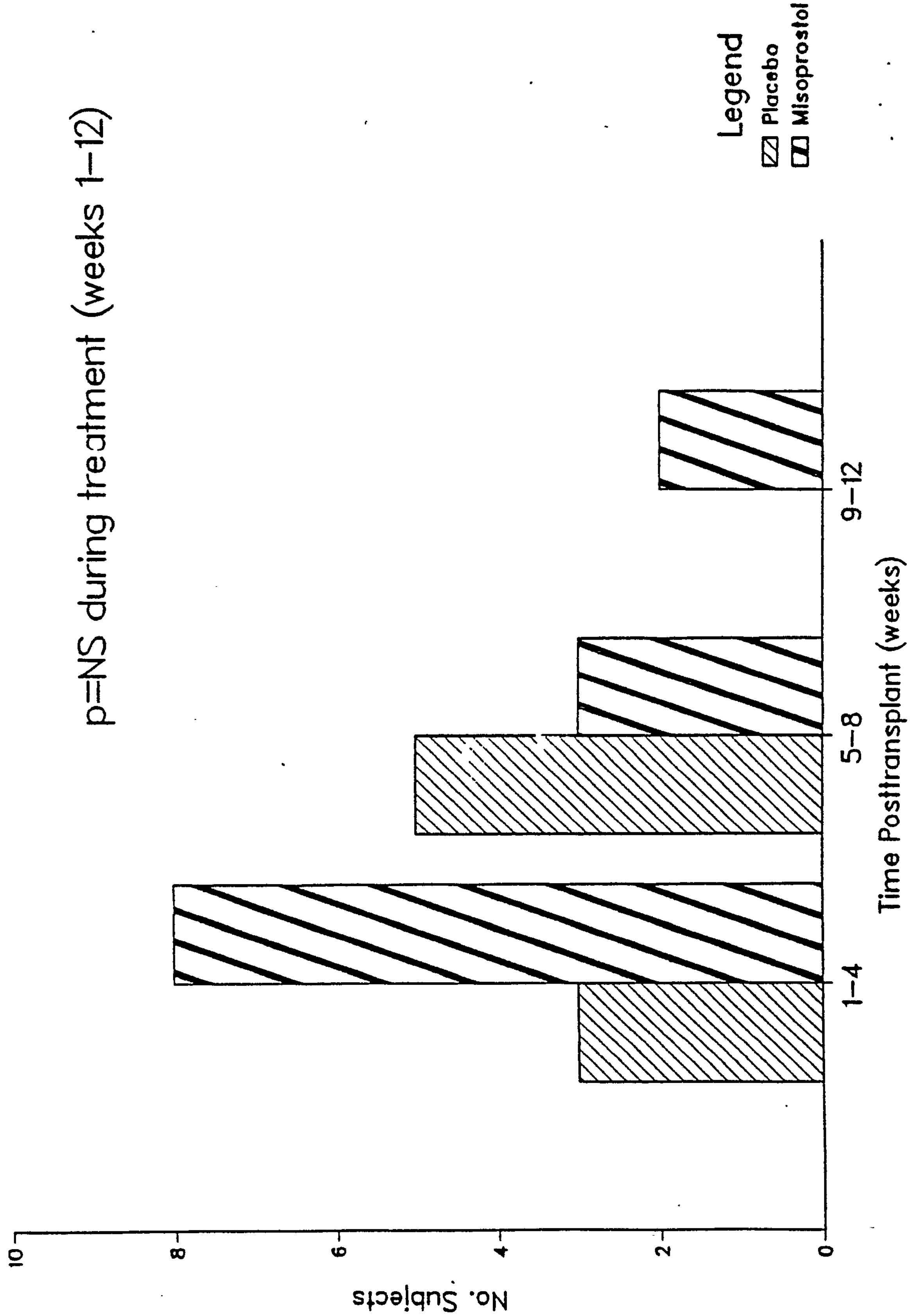
p=NS during treatment (weeks 1–12)
Legend
Placebo
Misoprostol
No. Subjects
0
2
4
6
8
10
1–4
5–8
9–12
Time Posttransplant (weeks)

FIGURE 6

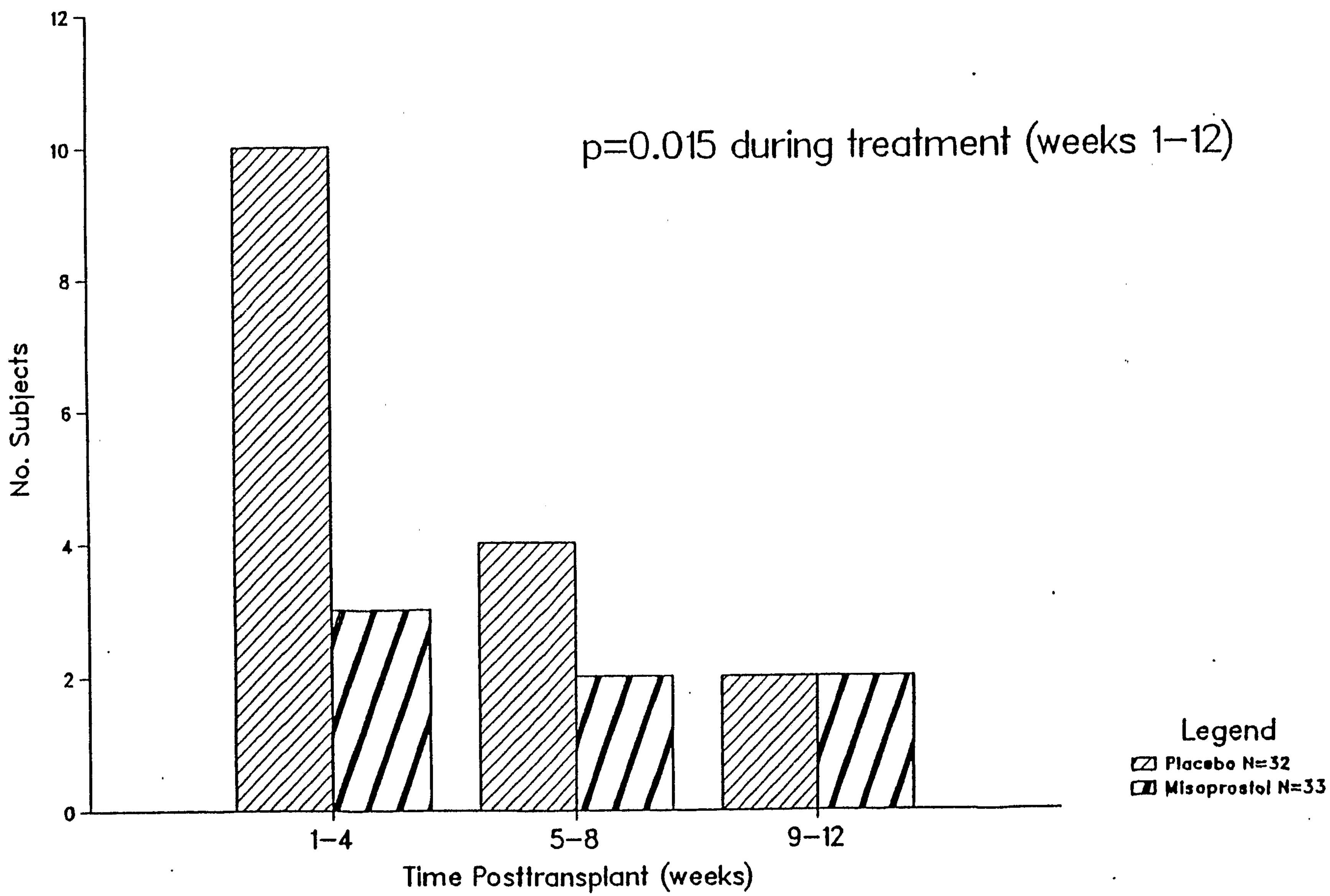
p=0.015 during treatment (weeks 1–12)
No. Subjects
0
2
4
6
8
10
12
1–4
5–8
9–12
Time Posttransplant (weeks)
Legend
Placebo N=32
Misoprostol N=33

FIGURE 7

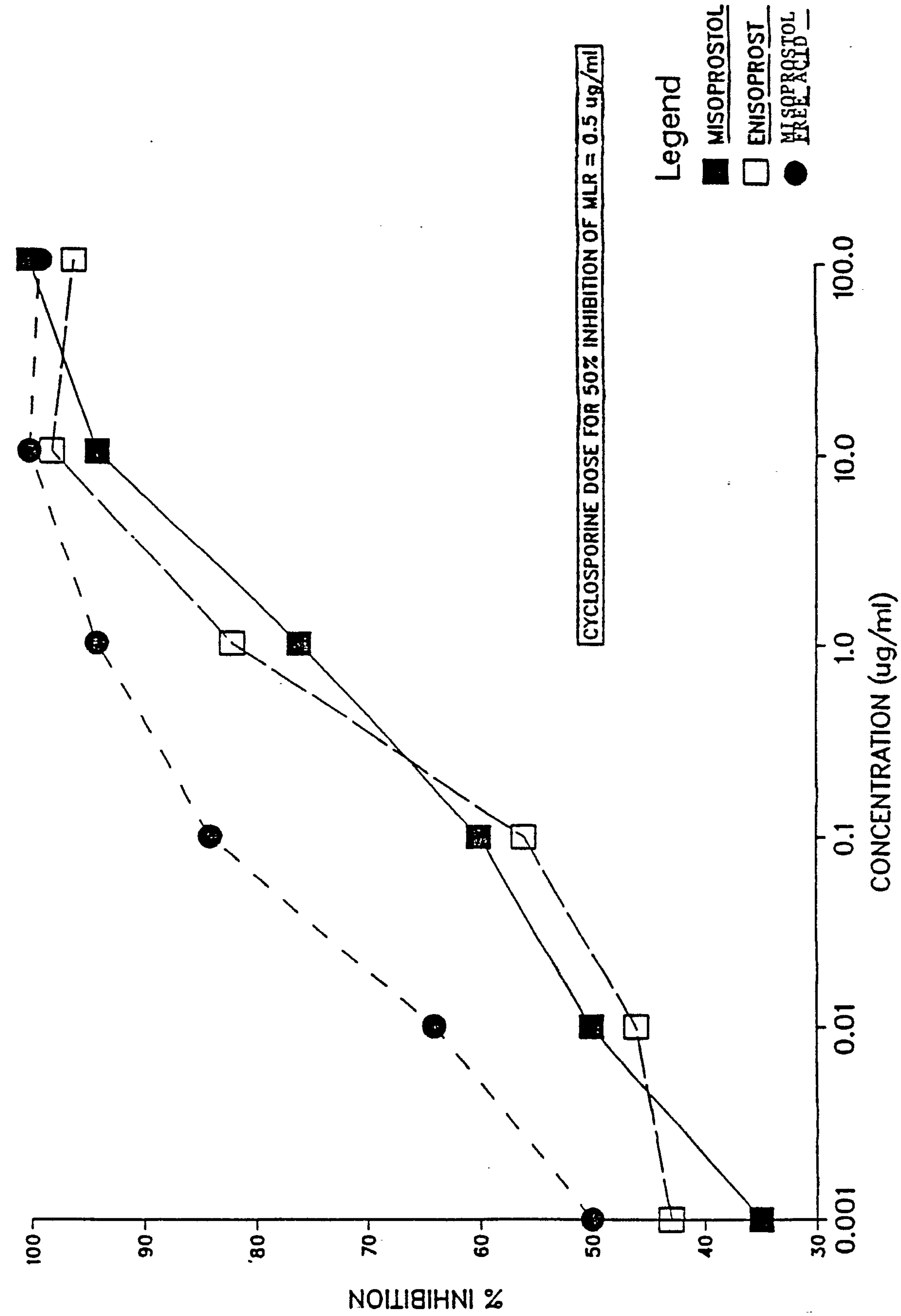
% INHIBITION
100
90
80
70
60
50
40
30
0.001
0.01
0.1
1.0
10.0
100.0
CONCENTRATION (ug/ml)
CYCLOSPORINE DOSE FOR 50% INHIBITION OF MLR = 0.5 ug/ml
Legend
MISOPROSTOL
ENISOPROST
MISOPROSTOL FREE ACID

FIGURE 8

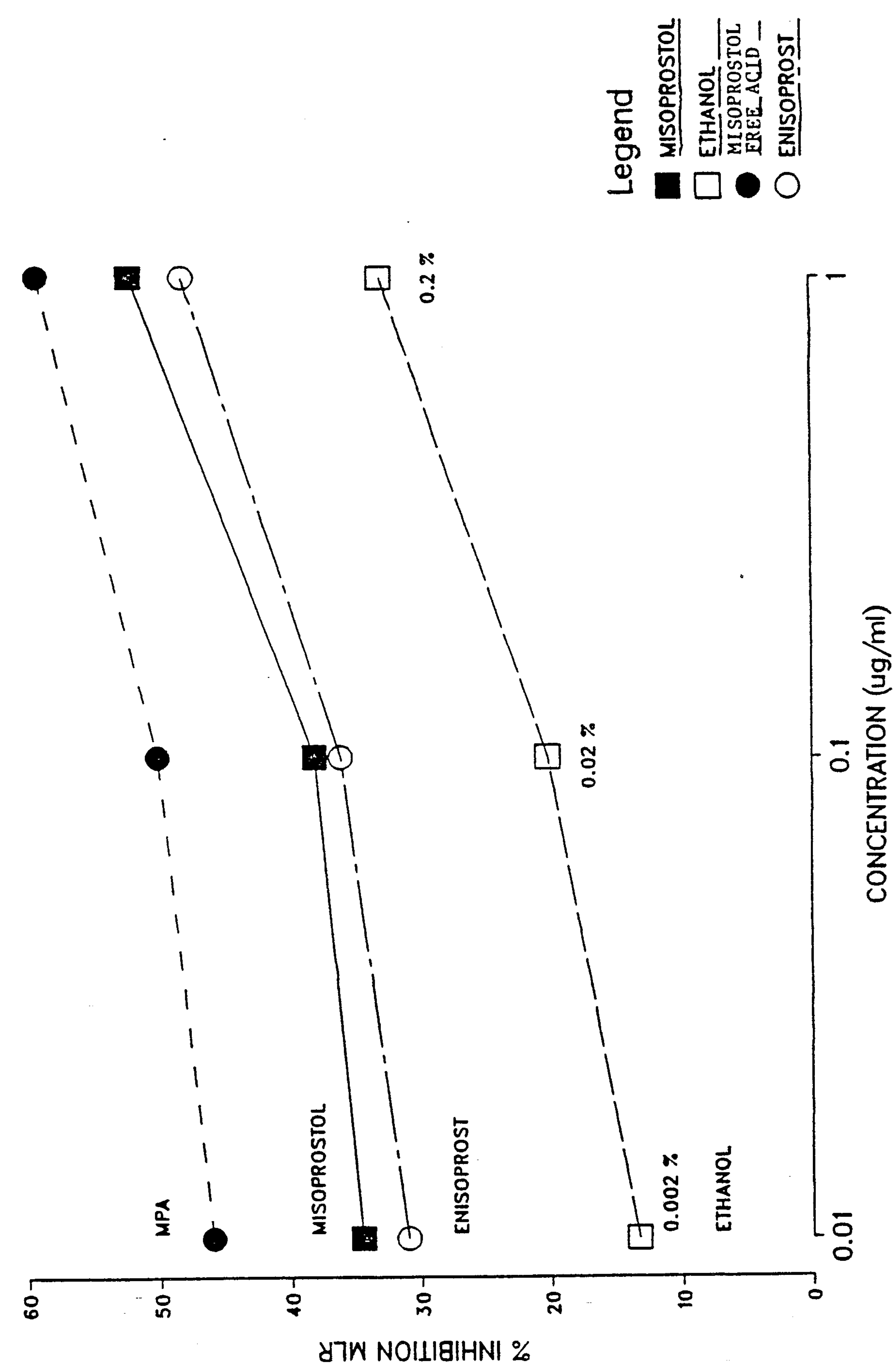
Legend
MISOPROSTOL
ETHANOL
MISOPROSTOL FREE ACID
ENISOPROST
MPA
MISOPROSTOL
ENISOPROST
ETHANOL
0.002 %
0.02 %
0.2 %
% INHIBITION MLR
0
10
20
30
40
50
60
0.01
0.1
1
CONCENTRATION (ug/ml)

FIGURE 9

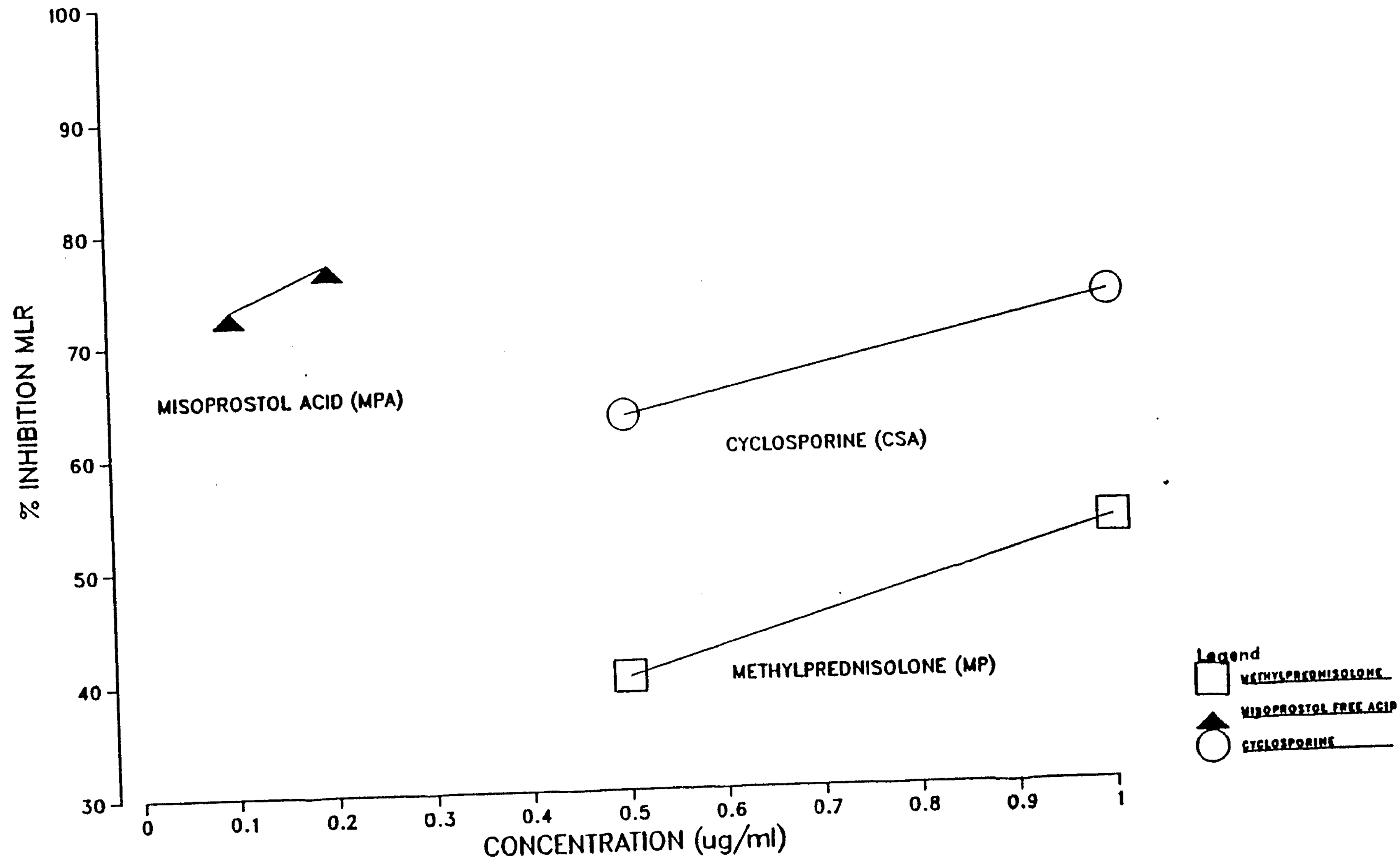
% INHIBITION MLR
100
90
80
70
60
50
40
30
0
0.1
0.2
0.3
0.4
0.5
0.6
0.7
0.8
0.9
1
CONCENTRATION (ug/ml)
MISOPROSTOL ACID (MPA)
CYCLOSPORINE (CSA)
METHYLPREDNISOLONE (MP)
Legend
METHYLPREDNISOLONE
MISOPROSTOL FREE ACID
CYCLOSPORINE

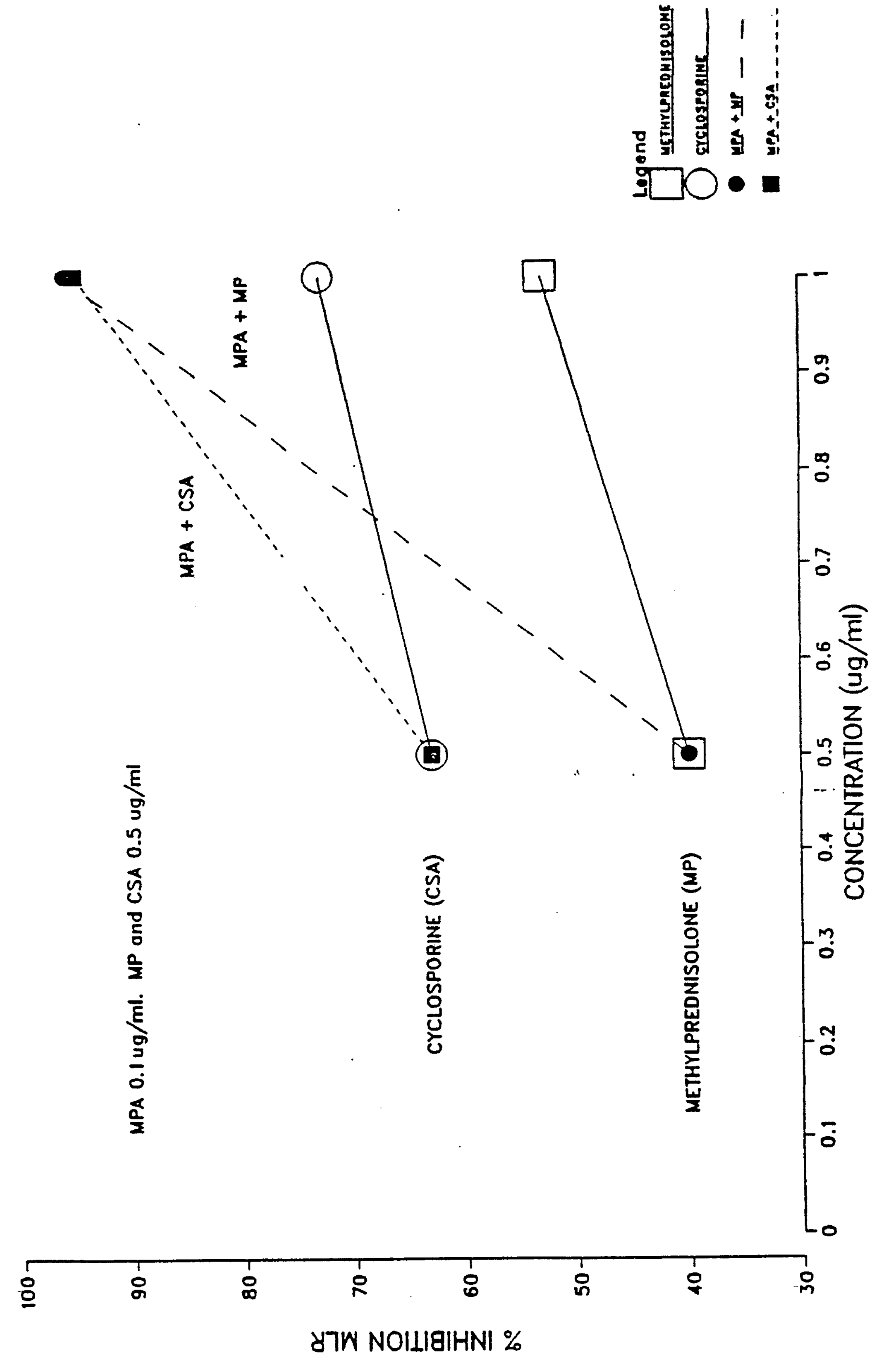
MPA 0.1 ug/ml. MP and CSA 0.5 ug/ml
CYCLOSPORINE (CSA)
METHYLPREDNISOLONE (MP)
MPA + CSA
MPA + MP
% INHIBITION MLR
100
90
80
70
60
50
40
30
CONCENTRATION (ug/ml)
0
0.1
0.2
0.3
0.4
0.5
0.6
0.7
0.8
0.9
1
Legend
METHYLPREDNISOLONE
CYCLOSPORINE
MPA + MP
MPA + CSA

FIGURE 10

European Patent Office

# EUROPEAN SEARCH REPORT

Application Number

EP 89 12 2544

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 705 092 (BAYER AG)<br>* Page 2, line 1 - page 5, line 40 (claims 1-11) *<br>--- | 1-20 | A 61 K 37/02 //<br>(A 61 K 37/02<br>A 61 K 31:557) |
| A | UNLISTED DRUGS, vol. 40, no 3, March 1988, Chatham, New Yersey, US, page 49f; "CSA-AZA-P"<br>--- | 1-20 | |
| A | CHEMICAL ABSTRACT, vol. 109, no 7, 15th August 1988, page 147, abstract no. 49104v, Columbus, Ohio, US; M.S. Paller: "Effects of the progstaglandin E1 analog misoprostol on cyclosporine nephrotoxity", & TRANSPLANTATION 1988, 45(6), 1126-31<br>* Abstracts *<br>----- | 1-20 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 12-02-1990 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)